# FASCICULE DE BREVET EUROPEEN

(11) **EP 3 765 161 B1**
(45) Date de publication et mention de la délivrance du brevet: **12.10.2022**
(21) Numéro de dépôt: 19716294.4
(22) Date de dépôt: 18.03.2019
(51) Int. Cl.: A61Q 19/02, A61K 8/9789

(54) **UTILISATION D'UN EXTRAIT D'ILEX AQUIFOLIUM POUR LE TRAITEMENT DES DESORDRES DE LA PIGMENTATION ET POUR LE BLANCHIMENT DE LA PEAU**
VERWENDUNG EINES EXTRAKTES VON ILEX AQUIFOLIUM ZUR BEHANDLUNG VON PIGMENTSTÖRUNGEN UND ZUM BLEICHEN DER HAUT
USE OF AN ILEX AQUIFOLIUM EXTRACT FOR THE TREATMENT OF PIGMENTATION DISORDERS AND FOR SKIN BLEACHING

(30) Priorité: 16.03.2018 FR 1870298
(43) Date de publication de la demande: 20.01.2021
(73) Titulaire: CENTRE NATIONAL DE LA RECHERCHE SCIENTIFIQUE, 75016 Paris (FR); Laboratoire In'Oya SAS, 13120 Gardanne (FR); Université d'Aix-Marseille, 13284 Marseille Cedex 07 (FR)
(72) Inventeur: MABROUK, Kamel, 13170 Les Pennes Mirabeau (FR); MKIMER, Laïla, 13580 La Fare-Les-Oliviers (FR); BENGELOUNE, Abd Haq, 13580 La Fare-les-Oliviers (FR); VERDONI, Marion, 83640 Plan-d'Aups-Sainte-Baume (FR); LUIS, José, 13385 Marseille Cedex 5 (FR); GIGMES, Didier, 13013 Marseille 13e (FR)
(74) Mandataire: Barbot, Willy
(86) Numéro de dépôt international: PCT/EP2019/000082
(87) Numéro de publication internationale: WO 2019/174786

(56) Documents cités:
- WO-A1-2008/105605
- WO-A1-2014/184340
- US-A1- 2015 104 485
- DATABASE GNPD [Online] MINTEL; 3 mars 2008 (2008-03-03), "Whitening Milk", XP002786270, Database accession no. 1077620
- DATABASE WPI Week 201771 Thomson Scientific, London, GB; AN 2017-67379N XP002786271, & CN 107 174 562 A (LIU C) 19 septembre 2017 (2017-09-19)
- DATABASE WPI Week 200903 Thomson Scientific, London, GB; AN 2009-A66754 XP002786272, & KR 2008 0029037 A (KOREAN COSMETICS CO LTD) 3 avril 2008 (2008-04-03)

## Description

### Domaine de l'invention

L'invention se rapporte à l'utilisation d'un extrait de graines de plantes du genre *Ilex* pour blanchir/dépigmenter la peau.

### Contexte de l'invention

L'apparition des taches d'hyperpigmentation est à l'origine d'un aspect non homogène de la couleur de la peau.

Ce phénomène est dû principalement à l'emballement du processus de la mélanogenèse, lequel se compose d'une succession de réactions enzymatiques. Dans ce processus de mélanogenèse, la tyrosinase est considérée comme l'enzyme « clé ». En effet, elle catalyse la première étape de la synthèse de la mélanine qui consiste en l'hydroxylation de l'acide aminé L-tyrosine en dihydroxyphenylalanine (L-DOPA ; activité monophénolase). Cette première étape constitue l'étape « limitante » de la mélanogenèse. La L-DOPA synthétisée, est ensuite oxydée en DOPAquinone sous l'action de la tyrosinase (activité diphénolase). En présence de composés thiolés, tels que le glutathion et/ou la cystéine, la voie de synthèse de phéomélanines (pigments jaunes - rouges) est favorisée. Lors de l'exposition solaire, et sous l'effet des rayonnements UV, les phéomélanines sont responsables de la formation de radicaux libres toxiques. En absence de ces composés thiolés, la DOPAquinone est convertie en DOPAchrome (un produit secondaire coloré rouge), il s'agit du précurseur de la voie de synthèse des eumélanines (pigments bruns-noirs). Lors de l'exposition solaire, les eumélanines jouent un rôle primordial dans la photoprotection naturelle. Les eumélanines peuvent ainsi absorber les radicaux libres produits dans les cellules par les radiations UV, empêchant que l'ADN soit endommagé. Au cours de la synthèse des eumélanines interviennent deux enzymes associés à la tyrosinase, lesquels sont appelés « tyrosinase related proteins » (TRP) : la TRP-1 et la TRP-2. La synthèse de mélanine dans le mélanocyte est principalement stimulée par la liaison de l'hormone mélanotrope ou α-Melanocyte-stimulating hormone (a-MSH) à son récepteur membranaire, melanocortin receptor (MC1R). En effet, suite à cette liaison une cascade de réactions a lieu dans les mélanocytes aboutissant à la transcription des gènes codant pour la tyrosinase, la TRP-1 et la TRP-2.

Chez les personnes à peau noire, les mélanosomes sont de grande taille, nombreux et complètement mélanisés. En effet la diffusion de mélanine y est plus importante, elle est présente jusqu'aux couches les plus superficielles (couche cornée chez les sujets les plus pigmentés). Cette répartition de la mélanine explique le fait que les peaux noires ont tendance à mal cicatriser, des traumatismes même minimes peuvent ainsi provoquer des hyperpigmentations de la peau. Un avantage lié à cette grande proportion de mélanine est une meilleure protection face au soleil.

Le processus de la mélanogénèse est soumis à de nombreux paramètres propres à l'organisme, mais également dépendants de facteurs extérieurs comme les rayons ultraviolets (UV). Les facteurs environnementaux, génétiques et/ou hormonaux peuvent moduler la quantité, le type et la distribution de la mélanine dans la peau, les cheveux, les yeux. Ainsi, même avec de légères modifications de l'état physiologique d'une personne ou de son exposition à des facteurs externes, cela peut affecter la couleur de la peau de manières transitoire (masque de grossesse dû à une surexpression d'hormones) ou permanente (apparition de taches de vieillesse).

Les causes d'apparition des désordres cutanés hyper-pigmentaires sont multiples, et définissent ainsi le type de taches. Ces troubles pigmentaires sont, en général, le résultat de mécanismes physiopathologiques divers, incluant des variations qualitatives ou quantitatives du pigment mélanique, mais également des anomalies de distribution de la mélanine. Le plus souvent, ces anomalies sont dues à un trouble de la mélanogénèse avec une stimulation prononcée de la synthèse de la mélanine au sein des mélanosomes, à un transfert plus important du pigment mélanique vers les kératinocytes ou encore à une accumulation de mélanine dans le derme. Ces taches d'hyperpigmentation représentent, pour les individus de phototype mate à noir, plus de 60 % des consultations chez un dermatologue. On distingue plusieurs types d'hyperpigmentations, avec les hyperpigmentations i) génétiques (ex. tâches café au lait, éphélides, nævus, lentigo, ...) ; ii) endocriniennes (ex. le mélasma ou le masque de grossesse) ; iii) carentielles (ex. pellagre dû à une carence de vitamine B3) ; iv) toxi-médicamenteuses (ex. minocyline) et v) post-inflammatoires.

Pour traiter ces problématiques d'hyperpigmentations, on utilise aujourd'hui la vitamine C et la vitamine E et leurs dérivés, les rétinoïdes, l'arbutine, l'hydroquinone, l'acide kogique, l'acide azélaïque, l'acide glycolique, le gluthation et ses dérivés. Ces composés présentent différents modes d'actions parmi lesquels l'inhibition de l'activité de la tyrosinase, la réduction de la quantité de mélanine formée ou encore la stimulation de l'élimination de la mélanine au sein des kératinocytes.

Maintenant, ces différents composés présentent un certain nombre d'inconvénients. Ainsi, l'hydroquinone dont l'utilisation a été interdite en Europe depuis 2001, mais qui reste autorisée dans d'autres pays parmi lesquels les Etats-Unis, présente une cytotoxicité non négligeable.

D'autres composés, tels que les dermocorticoïdes (ex. clobétasol propionate et béthamétasone dipropionate) sont à l'origine de dermatoses infectieuses. En outre, leur utilisation prolongée cause également des dommages sur la structure et la vascularisation de la peau. En Europe, ils sont donc uniquement autorisés sur prescription.

Les rétinoïdes bénéficient d'une efficacité prouvée dans le traitement du mélasma, mais présentent des effets secondaires tels que des brûlures, des érythèmes et une sécheresse de la peau. De ce fait, son utilisation et celle de ses dérivés dans les produits cosmétiques est interdite en Europe depuis 2009. Les mêmes effets secondaires sont observés avec l'acide azélaïque et l'acide glycolique. Maintenant, leur incorporation dans les produits cosmétiques est encore autorisée en Europe à ce jour.

Toujours en lien avec les composés utilisés pour traiter l'hyperpigmentation, on trouve encore le n-butylrésorcinol, ou rucinol, qui est un inhibiteur de l'activité de la TRP-1 mais dont l'application peut entraîner des brûlures, des érythèmes, une desquamation de la peau.

Finalement, l'ensemble de ces composés présente des effets secondaires non négligeables dont il résulte qu'il subsiste un besoin réel d'identifier de nouveaux agents dépigmentants ou blanchissants qui soient non seulement efficaces et stables dans le temps, mais plus encore d'une faible toxicité.

### Résumé de l'invention :

Les inventeurs ont identifié une activité d'inhibition de la tyrosinase associée à un extrait de graines *d'Ilex aquifolium* dont l'efficacité est avérée à une teneur d'un pourcent mais qui n'induit aucune cytotoxicité à une teneur de cinq pourcents.

Par le passé, différentes propriétés ont pu être associées à des plantes appartenant au genre *Ilex.*

Ainsi, les feuilles *d'Ilex purpura Haask* (*Ilex chinensis*) sont utilisées depuis des millénaires en médecine chinoise, notamment en lien avec les problèmes de gros intestins, de vessie ou encore de bronches. Il a pu être démontré qu'un extrait de ces feuilles inhibait notamment la Cyclooxygenase-2 (COX-2) et permettait d'envisager son utilisation comme anti-inflammatoire notamment pour éclaircir les zones de rougeur (cf. demande de brevet US 2005/0048140 A1).

Les feuilles d'*Ilex paraguariensis* sont utilisées de façon ancestrale pour préparer le maté qui joue un rôle d'excitant et qui comprend de la caféine. Maintenant, cet extrait est également considéré comme lipolytique, car il augmente la beta-oxydation des acides gras et abaisse ainsi l'obésité chez l'animal. Les feuilles *d'Ilex latifolia* présentent des propriétés similaires et ont montré des propriétés antioxydantes, abaissant les lipides, anti-inflammatoires, réduisant le poids, et anti-tumorales.

Il a en outre été décrit les propriétés antibactériennes et antifongiques des feuilles *d'Ilex aquifolium,* lesquelles peuvent être utilisées pour le traitement d'affections dermatologiques (cf. demande internationale WO2014/184340 A1).

Maintenant, rien ne laissait présager qu'un extrait de graines d'une plante appartenant au genre *Ilex* présentait également des propriétés dépigmentantes et/ou blanchissantes.

Tout au plus parmi les milliers de références en lien avec les propriétés du genre Ilex, on trouve uniquement la demande de brevet KR 2008 0029037 A qui décrit un extrait hydroalcoolique de branches broyées *d'Ilex rotunda* et la faible activité d'inhibition de la tyrosinase obtenu avec celui-ci.

Maintenant, ni ce document ni aucun autre ne suggère qu'une activité d'inhibition de la tyrosinase obtenu avec un extrait spécifiquement obtenu à partir de graines appartenant au genre *Ilex,* notamment un extrait glycériné et/ou un extrait provenant d'*Ilex aquifolium.*

Le document XP002786270 décrit une émulsion blanchissante pour la peau, inhibant la production de mélanine (§ description produit) et comprenant un extrait *d'Ilex paraguaynensis* (§ ingrédients).

La demande de brevet US 2015/104485 A1 décrit une composition comprenant au moins deux ingrédients actifs inhibant la mélanogénèse dont l'un est un peptide synthétisé chimiquement et l'autre peut notamment être un extrait d'*Ilex purpurea.*

La demande internationale PCT WO 2008/105605 A1 décrit l'utilisation d'un extrait de feuilles *Forsythia suspensa* (exemple 1 et 2) inhiber la mélanogénèse, mais envisage à titre subsidiaire l'utilisation d'un extrait *d'Ilex rotunda.*

L'invention porte donc sur une composition comprenant au moins un extrait de graines d'une plante choisies parmi *Ilex aquifolium, Ilex arnhemensis, Ilex canariensis, Ilex chinensis, Ilex crenata, Ilex mitis, Ilex opaca, Ilex paraguariensis, Ilex perado* et *Ilex vomitoria ;* lequel extrait est choisi parmi les extraits glycérinés, hydroglycérinés ou hydroalcoolique glycérinés et la teneur en extrait de graines d'une plante appartenant au genre *Ilex* dans la composition selon l'invention est comprise entre 0,5 et 5% en poids par rapport au poids total de la composition. A ce titre, cette composition est envisagée pour une utilisation dans le traitement et/ou la prévention de l'hyperpigmentation, laquelle hyperpigmentation est choisie parmi les hyperpigmentations post-inflammatoires, les hyperpigmentations avec un déterminisme génétique, les hyperpigmentations d'origine métabolique ou médicamenteuse ou tout autre lésion hyperpigmentaire.

L'invention porte également sur l'utilisation non-thérapeutique d'au moins un extrait de graines d'une plante appartenant au genre *Ilex* comme agent cosmétique pour le blanchiment/la dépigmentation de la peau.

L'invention porte enfin sur une méthode non-thérapeutique pour blanchir/dépigmenter la une zone peau humaine comprenant une étape d'application topique d'une quantité efficace sur ladite zone de peau ciblée d'un sujet d'une composition comprenant une quantité efficace d'un extrait de graines d'une plante appartenant au genre *Ilex.*

### Description des figures :

La figure 1 montre le pourcentage d'inhibition de mélanine obtenu avec un extrait de graines *d'Ilex aquifolium.*
La figure 2 montre la toxicité cellulaire d'une concentration croissante en extrait de graines *d'Ilex aquifolium.*

### Description détaillée de l'invention :

En premier lieu, l'invention est relative à une composition qui comprend au moins un extrait de graines d'une plante appartenant au genre *Ilex.*

Ce genre (*Ilex*) comprend plus de 400 espèces réparties dans le monde entier. Maintenant, si on trouve une grande diversité d'espèces en Amérique et dans le sud-est asiatique, une seule espèce est présente en Europe (*Ilex aquifolium,* nommé houx en français), en Afrique (*Ilex mitis*) ou encore en Australie (*Ilex arnhemensis*)*.* Ces plantes sont pour la plupart des plantes ligneuses, arbres ou arbustes, dont les feuilles sont généralement persistantes, et parfois munies de piquants. Les fleurs sont petites, blanches ou de couleur pâle, fréquemment rassemblées en petits bouquets à l'aisselle des feuilles. Enfin, les fruits sont des baies, d'un rouge éclatant chez presque toutes les espèces.

Maintenant, et préférentiellement, on utilisera des extraits de graines *d'Ilex aquifolium.*

Le terme « extrait » fait référence à une substance extraite d'un produit naturel, indépendamment de sa méthode d'extraction ou de la composition des ingrédients. Les procédés d'extraction sont bien connus de l'homme du métier et consistent typiquement en une macération des graines, broyées ou non, dans le solvant d'extraction.

De préférence, l'extrait de graines d'une plante appartenant au genre *Ilex* est un extrait glycériné.

De préférence, la teneur en extrait de graines d'une plante appartenant au genre *Ilex* dans la composition selon l'invention est comprise entre 1 et 3% en poids par rapport au poids total de la composition.

Selon un mode de réalisation préféré, la composition selon l'invention comprend également au moins un agent de desquamation. Cet agent vise en accélérant le renouvellement de la peau à accélérer simultanément son blanchiment apparent.

Les agents de desquamation utilisables peuvent être identifiés aisément par l'homme du métier. A titre d'exemple, on peut citer les α-hydroxyacides (ex. acide citrique, acide lactique, acide glycolique, acide mandélique, acide malique et acide tartrique), les β-hydroxyacides (ex. acide salicylique et ses dérivés), les α-cétoacides et les β-cétoacides, les rétinoïdes et en particulier le rétinol et les esters de rétinyle, les inhibiteurs d'HMG-CoA réductase, et les dérivés de sucre tels que le O-octanoyl-6'-β-D-maltose. De façon préférentielle, on utilisera un β-hydroxyacide, notamment l'acide salicylique ou l'un de ses dérivés.

Typiquement, la teneur en agent de desquamation dans la composition selon l'invention est comprise entre 0,01 et 10% en poids par rapport au poids total de la composition, de préférence entre 0,1 et 5%.

Selon un autre mode de réalisation préféré, la composition selon l'invention comprend également au moins un autre inhibiteur de la mélanogénèse. Cet autre inhibiteur de la mélanogénèse a vocation à augmenter encore l'action dépigmentante/blanchissante de la composition selon l'invention.

Les inhibiteurs de la mélanogénèse utilisables dans la composition selon l'invention peuvent être identifiés aisément par l'homme du métier et comprennent notamment l'acide kojique ; l'acide ellagique ; l'arbutine et ses dérivés tels que ceux décrits dans les demandes de brevet EP 895 779 et EP 524 109 ; l'hydroquinone ; les dérivés d'aminophénol tels que ceux décrits dans les demandes internationales WO 99/10318 et WO 99/32077 (ex. le N-cholestéryloxycarbonyl-para-aminophénol et le N-éthyloxycarbonyl-para-aminophénol); les dérivés d'iminophénol, en particulier ceux décrits dans la demande internationale WO 99/22707 ; l'acide L-2-oxothiazolidine-4-carboxylique ou procystéine, ainsi que ses sels et esters; l'acide ascorbique et ses dérivés, notamment le glucoside d'ascorbyle ; et les extraits de plantes, en particulier de réglisse, de mûrier et de scutellaire, sans que cette liste soit limitative.

A titre d'inhibiteur de la mélanogénèse, on préférera les inhibiteurs qui suivent, qu'ils soient seuls ou en combinaison :
∘ (1) un extrait de son de blé (INCI : *Triticum aestivum* extract, AXOLIGHT, GIVAUDAN). Typiquement, un tel extrait est utilisable à une teneur comprise 1 et 10% en poids par rapport au poids total de la composition, de préférence entre 1 et 5% et, de manière particulièrement préférée, comprise entre 2 et 3%.
∘ (2) l'oligopeptide-68 qui est un peptide de 12 acides aminés agoniste du TGF-β inhibiteur du facteur de transcription MITF. L'oligopeptide-68 est notamment disponible chez LUCAS MEYER (β-WHITE). Typiquement, la teneur en oligopeptide-68 comprise 0,5 et 5% en poids par rapport au poids total de la composition et, de préférence, entre 2 et 3%.
∘ (3) du nicotinamide, aussi connu sous le nom de niacinamide et qui est un dérivé de l'acide nicotinique. Typiquement, la teneur en nicotinamide est comprise 2 et 6% en poids par rapport au poids total de la composition et, de préférence, entre 3 et 5%.
∘ (4) la N-acétylglucosamine (N-acétyl-D-glucosamine ou GlcNAc ou NAG) qui est un monosaccharide ou ose modifié partageant le même squelette que le glucose. Typiquement, la teneur en N-acétylglucosamine comprise 1 et 6% en poids par rapport au poids totaal de la composition et, de préférence, entre 3 et 5%.
∘ (5) la L-Arginine. Typiquement, la teneur en L-Arginine comprise 0,1 et 3% en poids par rapport au poids total de la composition et, de préférence, entre 0,2 et 0,5%.

Plus généralement, la teneur en inhibiteur de la mélanogénèse dans la composition selon l'invention, et sans considérer la teneur en extrait de graines d'une plante appartenant au genre *Ilex,* est comprise entre 0,001 et 20% en poids par rapport au poids total de la composition, de préférence entre 1 et 15%.

Maintenant, et pour ce qui est de la composition selon l'invention, il s'agit typiquement d'une composition topique qui est applicable sur n'importe quelle zone de peau du corps, à l'exclusion naturellement des membranes muqueuses (yeux, bouche, etc.) et qui est dénommée MEL'OYA^{™}. A ce titre, cette composition (MEL'OYA^{™}) peut se présenter sous toutes les formes galéniques normalement utilisées pour ce type d'application, notamment sous forme d'une solution aqueuse ou huileuse, d'une émulsion huile-dans-eau ou eau-dans-huile ou multiple, d'une émulsion siliconée, d'une microémulsion ou nanoémulsion, d'un gel aqueux ou huileux ou d'un produit anhydre liquide, pâteux ou solide.

Cette composition (MEL'OYA^{™}) peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

De façon connue, cette composition (MEL'OYA^{™}) peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les chélateurs, les charges, les filtres, les pigments, les huiles essentielles, les absorbeurs d'odeur, les matières colorantes ou tout autre actif cosmétique (actif anti-âge, raffermissant, restructurant, énergisant, anti-ride, décontractant, hydratant, antimicrobien, séborrégulateur, purifiant, apaisant, relaxant, anti-stress, immunomodulateur, liftant, repulpant, etc....). Les teneurs de ces différents adjuvants sont celles classiquement utilisées dans les domaines considérés, et par exemple de 0,01 à 20 % en poids du poids total de la composition. Ces adjuvants, selon leur nature, peuvent être introduits dans la phase grasse ou dans la phase aqueuse. Maintenant, ces adjuvants, ainsi que leurs concentrations, doivent être tels qu'ils ne nuisent pas aux propriétés avantageuses d'un extrait de graines d'une plante appartenant au genre *Ilex.*

Selon encore un autre mode de réalisation préféré de l'invention, la composition (MEL'OYA^{™}) selon l'invention pourra comprendre en outre au moins un filtre UVA et/ou UVB choisi parmi les filtres organiques et les filtres inorganiques.

Des exemples de filtres UVA organiques adaptés comprennent notamment :
∘ (1) les dérivés de benzophénone, les benzophénones 3 et 5 étant préférées ;
∘ (2) les dérivés de triazine, et en particulier la 2,4-bis {[4-(2-éthyl-hexyloxy)-2-hydroxy]-phényl}-6-(4-méthoxy-phényl)-1,3,5-triazine disponible auprès de la société CIBA GEIGY sous la dénomination commerciale TINOSORB S et le 2,2'-méthylènebis-[6-(2H benzotriazol-2-yl)4-(1,1,3,3-tétraméthylbutyl)phénol] disponible auprès de la société CIBA GEIGY sous la dénomination commerciale TINOSORB M ;
∘ (3) l'acide téréphtalylidène dicamphre sulfonique ou acide benzène 1,4 [di(3-méthylidènecampho 10-sulfonique)]

A titre d'exemple de filtres UVB organiques, on peut notamment citer :
∘ (1) les dérivés de l'acide salicylique, en particulier le salicylate d'homomenthyle et le salicylate d'octyle ;
∘ (2) les dérivés de l'acide cinnamique, en particulier le p-méthoxycinnamate de 2-éthylhexyle;
∘ (3) les dérivés de β,β'-diphénylacrylate liquides, en particulier l'α-cyano-α,β' diphénylacrylate de 2-éthylhexyle, ou octocrylène ;
∘ (4) les dérivés de l'acide p-aminobenzoïque ;
∘ (5) le 4-méthyl benzylidène camphre;
∘ (6) l'acide 2-phénylbenzimidazole 5-sulfonique ; et
∘ (7) les dérivés de 1,3,5-triazine, en particulier la 2,4,6-tris[p-(2'-éthylhexyl-1'-oxycarbonyl)anilino]-1,3,5-triazine.

Les filtres inorganiques utilisables dans la composition selon l'invention sont en particulier les nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Lorsque la composition (MEL'OYA^{™}) selon l'invention prend la forme d'une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition. Comme matières grasses utilisables, on peut utiliser les huiles et notamment les huiles minérales (huile de vaseline), les huiles d'origine végétale (huile d'avocat, huile de soja), les huiles d'origine animale (lanoline), les huiles de synthèse (octyldodécanol), les huiles siliconées (cyclométhicone et diméthicone) et les huiles fluorées (perfluoropolyéthers). On peut aussi utiliser comme matières grasses des alcools gras tels que l'alcool cétylique, des acides gras tels que l'acide stéarique, des cires et des gommes et en particulier les gommes de silicone. Comme émulsionnants et coémulsionnants utilisables, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales TWEEN^{®} 20 ou TWEEN^{®} 60, par exemple ; les esters de sucre tels que le méthyl glucose sesquistéarate et/ou son dérivé oxyéthyléné ; et leurs mélanges. Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

En deuxième lieu, l'invention vise aussi une telle composition (MEL'OYA^{™}) pour une utilisation en tant que médicament et, plus spécifiquement pour le traitement et/ou la prévention de l'hyperpigmentation chez un sujet, laquelle hyperpigmentation est choisie parmi les hyperpigmentations post-inflammatoires, les hyperpigmentations avec un déterminisme génétique, les hyperpigmentations d'origine métabolique ou médicamenteuse ou tout autre lésion hyperpigmentaire.

La composition (MEL'OYA^{™}) visée est alors telle que décrite précédemment.

Le terme sujet vise lui un humain qui peut tout aussi bien être un homme qu'une femme.

De préférence, le sujet en question présente un phototype compris entre IV et VI. Les sujets en question présentent graduellement, une peau mate, une peau foncée ou une peau noire.

Le terme « traiter » signifie soigner un état de maladie déjà présent chez un patient ou un sujet. En lien avec cette notion de traiter, celle-ci peut inclure l'arrêt du développement de la maladie (stabilisation) ou l'amélioration de l'état du patient avec une régression des symptômes.

Le terme « prévenir », signifie empêcher ou retarder l'apparition des symptômes de la maladie, et ceci de manière complète ou partielle.

Le terme « hyperpigmentation » se réfère à une gamme de troubles cutanés causés par une augmentation de la production de mélanine et résultant en une augmentation de la pigmentation cutanée dans des régions localisées. L'hyperpigmentation se réfère à une hyperpigmentation post-inflammatoire due à une abrasion, à une brulure, à une cicatrice (hyperpigmentation cicatricielle), à une dermatose, ou encore à une allergie de contact, ou encore une hyperpigmentation d'origine métabolique ou médicamenteuse.

En troisième lieu, l'invention vise une utilisation non-thérapeutique d'au moins un extrait de graines d'une plante appartenant au genre *Ilex* comme agent cosmétique pour le blanchiment/la dépigmentation de la peau.

Cet agent cosmétique est utilisé au sein d'une composition selon l'invention. A ce titre, les différentes préférences en liant avec l'extrait utilisé sont telles que décrites précédemment.

Par « agent de blanchiment de la peau », on fait référence à tout composé ou substance qui a l'effet d'altérer la pigmentation de la peau et qui présente une activité anti-tyrosinase et/ou une activité anti-mélanogénique.

Par « agent de dépigmentation de la peau », on fait référence à la réduction de la pigmentation de la peau qui existe déjà et/ou à la prévention de toute pigmentation additionnelle supérieure à la pigmentation naturelle. Par exemple, la dépigmentation est obtenue en réduisant la formation ou le niveau de formation de mélanine.

De manière préférentielle, l'utilisation de l'agent cosmétique pour blanchir/dépigmenter de l'invention sert à empêcher l'apparition des signes de vieillissement cutané photo-induit ou chronologique et/ou à les atténuer. L'expression « les signes de vieillissement cutané photo-induits » fait référence au vieillissement extrinsèque de la peau causé par le soleil et, plus particulièrement, par les rayons ultraviolets qui entrainent une augmentation du taux de radicaux libres avec un important stress oxydatif au niveau du derme. En outre, l'expression « les signes du vieillissement chronologiques » fait référence au vieillissement intrinsèque de la peau qui conduit à une atrophie progressive et à une dégénérescence du derme, de l'hypoderme et des structures de soutien de la peau.

Enfin, l'invention vise une méthode non-thérapeutique pour blanchir/dépigmenter une zone de peau humaine comprenant une étape d'application sur la zone de peau ciblée d'un sujet d'une composition telle que décrite précédemment.

L'expression « quantité efficace » d'une composition contenant un agent actif signifie une quantité suffisante de cette composition pour fournir l'effet désiré. En l'espèce, l'effet est le blanchiment/la dépigmentation de la zone de peau ciblée.

Plus spécifiquement, cette méthode pour blanchir/dépigmenter peut viser à empêcher l'apparition des signes de vieillissement cutané photo-induit ou chronologique et/ou à les atténuer.

Les expériences suivantes sont fournies pour illustrer les réalisations de la l'invention et ne doivent pas être considérées comme limitant la portée de l'invention.

### Exemples

### 1) L'extrait de graines d'Ilex aquifolium est un inhibiteur de la mélanogénèse

Dans le cadre de leurs recherches, les inventeurs ont testé l'effet sur la mélanogénèse d'un grand nombre de composants parmi lesquels un extrait glyceriné d'*Ilex Aquifolium.*

L'effet sur la mélanogénèse des différents composants, dont cet extrait, ont été testés sur des cellules issues de mélanomes humains, appelées les M₄Be, qui ont été obtenues par le Centre Léon Bérard (CRCL, INSERM U1052, CNRS U5286). Ces cellules ont été cultivées dans du milieu Mac Coy (GIBCO) supplémenté avec 10 % de sérum de veau fœtal (Lonza), une solution d'acides aminés non essentiels IX (GIBCO), une solution de pénicilline/streptomycine IX (GIBCO), une solution de L-Glutamine IX (GIBCO). Les cellules sont incubées en atmosphère humide à 37 °C avec 5 % de CO2.

Les cellules sont ensemencées à 5.10⁴ cellules/puits dans une plaque P24 puits et stimulées par 100 nM d' α-MSH, l'hormone stimulant la production de mélanine. 24 h plus tard, les cellules sont traitées avec différents composants parmi lesquels un milieu de culture comprenant 1% (p/p) d'un extrait glyceriné de graines *d'Ilex Aquifolium* et du Calcium D-Pantéthéine-S-Sulfonate (PaSSO₃Ca ; SOGO PHARMACEUTICAL CO) à 265 µM en tant que contrôle positif. Un contrôle négatif ne contenant aucun composant en plus du milieu de culture a également été utilisé, ainsi qu'une lyse des cellules à la soude (NaOH). Après 48 heures, la quantité de mélanine secrétée dans le milieu est mesurée spectrophotométriquement à 405 nm et normalisée à la quantité de protéines.

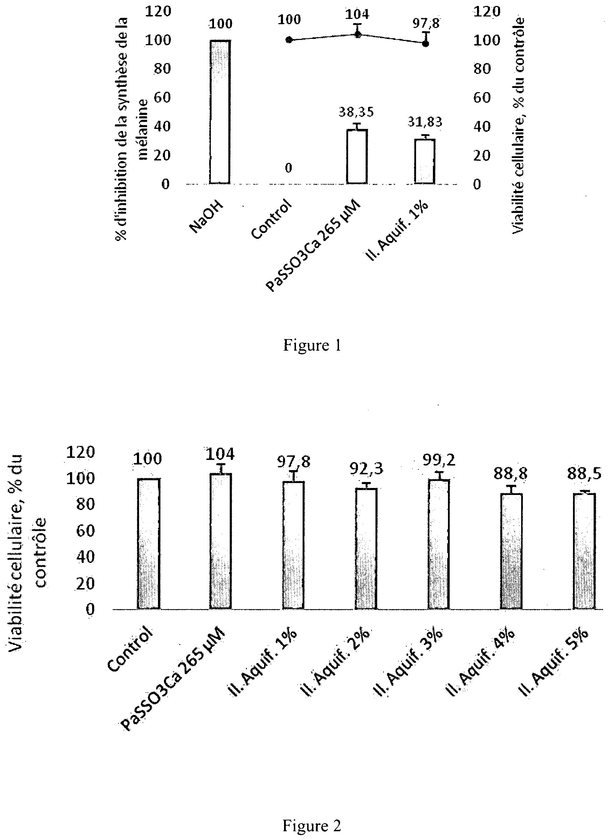

La figure 1 montre la production de mélanine en présence de PaSSO₃Ca, de 1% d'un extrait glycériné de graines *d'Ilex aquifolium,* ou de milieu de culture seul.

Les résultats montrent qu'un extrait glycériné de graines *d'Ilex Aquifolium* utilisé à 1% induit un pourcentage d'inhibition de la synthèse de la mélanine de l'ordre de 32 % sur les cellules de la lignée M4Be. D'ailleurs, cette inhibition se révèle proche de celle obtenue avec le contrôle positif. A noter que les mêmes expériences réalisées cette fois avec des extraits aqueux et hydro-alcoolique de graines *d'Ilex Aquifolium* ont montré également une capacité d'inhibition de la synthèse de la mélanine dans ces mêmes cellules, même si ces valeurs d'inhibition se sont révélées inférieure à celle obtenue avec l'extrait glycériné.

### 2) L'extrait de graines d'Ilex aquifolium

En vue de préciser les propriétés d'inhibition de la mélanogénèse d'Ilex aquifolium, d'autres parties de la plante sont testées.

Dans ce cadre, et en plus des extraits de graines, des extraits de feuilles, de branches et de racines sont réalisés.

Comme précédemment, ces extraits sont testés sur des cellules M₄Be.

Ces expériences montrent que l'extrait de graines *d'Ilex Aquifolium* induit un pourcentage d'inhibition de la synthèse de la mélanine bien supérieur à celui obtenu avec les extraits de feuilles, de branches ou de racines.

### 3) L'activité d'inhibition de la mélanogenèse est présentes dans

En vue de préciser les propriétés d'inhibition de la mélanogénèse par des plantes du genre Ilex, d'autres espèces sont testées.

Dans ce cadre, et en plus des extraits de graines *d'Ilex* aquifolium, mais aussi d'*Ilex arnhemensis, d'Ilex canariensis, d'Ilex chinensis, d'Ilex crenata, d'Ilex mitis, d'Ilex opaca,* d'*Ilex paraguariensis,* d'*Ilex perado* et *d'Ilex vomitoria* sont réalisés.

Comme précédemment, ces extraits sont testés sur des cellules M₄Be.

Ces expériences montrent que l'activité d'inhibition de la mélanogenèse, si elle est présente en association avec l'extrait de graines *d'Ilex aquifolium* l'est aussi avec les extraits de graines des autres espèces d'Ilex testées.

### 4) L'extrait de graines d'Ilex aquifolium ne présente qu'une faible cytotoxicité

La cytotoxicité de l'extrait de graines *d'Ilex Aquifolium* sur les cellules de la lignée M4Be a ensuite été évaluée par le test MTT qui est une méthode rapide de numération des cellules vivantes. Dans le détail, le réactif utilisé est le sel de tétrazolium MTT (bromure de 3-(4,5-dimethylthiazol-2-yl)-2,5-diphenyl tétrazolium) dont l'anneau de tétrazolium est réduit en formazan par la succinate déshydrogénase mitochondriale des cellules vivantes actives. Le formazan obtenu forme un précipité dans la mitochondrie de couleur violette. Ainsi, la quantité de précipité formée est directement proportionnelle à la quantité de cellules vivantes.

Pour ce test MTT, 25 000 cellules M₄Be ont été ensemencées par puits dans une plaque de 96 puits et incubées dans des conditions de culture standards. Après 24 h de croissance, le surnageant a été éliminé et remplacé par un milieu de culture frais contenant diverses concentrations d'extrait glycériné d'*Ilex Aquifolium* (1, 2, 3, 4 et 5 %). Après 6 h d'incubation à 37 ° C, le surnageant a été retiré et remplacé par 50 µL de solution de MTT à 0,5 m/ml. Les cellules ont ensuite été incubées avec la solution de MTT pendant 2 h avant d'effectuer un lavage avec une solution de PBS 1×. Enfin, les cristaux de formazan contenus dans les puits ont été solubilisés par ajout de 100µL d'une solution de DMSO. Après homogénéisation, l'absorbance était mesurée à 600 nm.

La figure 2 montre, au regard des valeurs d'absorbance obtenues à 600 nm, les pourcentages de viabilité cellulaire dans les différentes conditions de culture.

Il apparaît que l'extrait glycériné *d'Ilex Aquifolium* utilisé jusqu'à 5% n'induit aucune mortalité cellulaire. Cela implique qu'à 1%, pourcentage que les auteurs souhaitent incorporer dans leur formulations cosmétiques, aucune mort cellulaire ne sera observée.

## Revendications

1. Une composition d'au moins un extrait de graines d'une plante choisie parmi *Ilex aquifolium, Ilex arnhemensis, Ilex canariensis, Ilex chinensis, Ilex crenata, Ilex mitis, Ilex opaca, Ilex paraguariensis, Ilex perado* et *Ilex vomitoria* ; lequel extrait est choisi parmi les extraits glycérinés, hydroglycérinés et hydroalcoolique glycérinés ; et la teneur en extrait de graines d'une plante appartenant au genre *Ilex* dans la composition est comprise entre 0,5 et 5% en poids par rapport au poids total de la composition.

2. La composition selon la revendication 1, **caractérisée en ce que** la plante du genre *Ilex est Ilex aquifolium.*

3. La composition selon l'une quelconque des revendications 1 ou 2, **caractérisée en ce que** l'extrait de graines d'une plante appartenant au genre *Ilex* est un extrait glycériné.

4. La composition selon l'une quelconque des revendications 1 à 3, **caractérisée en ce que** la teneur en extrait de graines d'une plante appartenant au genre *Ilex* dans la composition est comprise entre 1 et 3% par rapport au poids total de la composition

5. La composition selon l'une quelconque des revendications 1 à 4, **caractérisée en ce qu'**elle comprend également au moins un agent de desquamation et/ou au moins un autre inhibiteur de la mélanogénèse.

6. Une composition telle que définie à l'une quelconque des revendications 1 à 5 pour une utilisation dans le traitement et/ou la prévention d'une hyperpigmentation chez un sujet, laquelle hyperpigmentation est choisie parmi les hyperpigmentations post-inflammatoires, les hyperpigmentations avec un déterminisme génétique, les hyperpigmentations d'origine métabolique ou médicamenteuse ou tout autre lésion hyperpigmentaire.

7. Utilisation non-thérapeutique d'une composition comprenant au moins un extrait de graines d'une plante choisie parmi Ilex aquifolim, Ilex arnhemensis, Ilex canariensis, Ilex chinensis, Ilex crenata, Ilex mitis, Ilex opaca, Ilex paraguariensis, Ilex perado et Ilex vomitaria ; lequel extrait est choisi parmi les extraits glycérinés, hydroglycérinés et hydroalcoolique glycérinés ; la teneur dudit extrait est comprise entre 0,5 et 5% en poids par rapport au poids total de la composition, comme agent cosmétique pour le blanchiment/la dépigmentation de la peau.

8. L'utilisation selon la revendication 7 **caractérisée en ce que** ledit agent vise à prévenir et/ou traiter les signes de vieillissement cutané photo-induits ou chronologiques.

9. Une méthode non-thérapeutique pour blanchir/dépigmenter une zone de peau humaine comprenant une étape d'application topique d'une quantité efficace sur ladite zone de peau ciblée d'un sujet de la composition telle que définie à l'une quelconque des revendications 1 à 5.

## Patentansprüche

1. Zubereitung mindestens eines Extraktes von Samen einer Pflanze, ausgewählt unter *Ilex aquifolium, Ilex arnhemensis, Ilex canariensis, Ilex chinensis, Ilex crenata, Ilex mitis, Ilex opaca, Ilex paraguariensis, Ilex perado* und *Ilex vomitoria,* wobei der genannte Extrakt unter den Glyzerin-, Hydroglyzerin- und Glyzerin-Hydroalkoholextrakten ausgewählt wurde, und der Gehalt an Extrakt von Samen einer Pflanze der Gattung *Ilex* in der Zubereitung zwischen 0,5 und 5 Gewichtsprozent bezogen auf das Gesamtgewicht der Zubereitung liegt.

2. Zubereitung nach Patentanspruch 1, **dadurch gekennzeichnet, dass** es sich bei der Pflanze der Gattung *Ilex* um *Ilex aquifolium* handelt.

3. Zubereitung nach irgendeinem der Patentansprüche 1 oder 2, **dadurch gekennzeichnet, dass** der Extrakt von Samen einer Pflanze der Gattung *Ilex* ein Glyzerinextrakt ist.

4. Zubereitung nach irgendeinem der Patentansprüche 1 bis 3, **dadurch gekennzeichnet, dass** der Gehalt an Extrakt von Samen einer Pflanze der Gattung *Ilex* in der Zubereitung zwischen 1 und 3 Gewichtsprozent bezogen auf das Gesamtgewicht der Zubereitung liegt.

5. Zubereitung nach irgendeinem der Patentansprüche 1 bis 4, **dadurch gekennzeichnet, dass** sie ebenfalls mindestens ein Abschuppungsmittel und/oder mindestens einen anderen Melanogenesehemmer enthält.

6. Zubereitung nach irgendeinem der Patentansprüche 1 bis 5 für den Gebrauch in der Behandlung und/oder Vorbeugung einer Hyperpigmentierung bei einer Person, wobei diese Hyperpigmentierung unter postinflammatorischer Hyperpigmentierung, genetisch bedingter Hyperpigmentierung, stoffwechselbedingter oder medikamentös bedingter Hyperpigmentierung oder jeglicher anderen Hyperpigmentierungsläsion ausgewählt wurde.

7. Nicht-therapeutischer Gebrauch einer Zubereitung, die mindestens einen Extrakt von Samen einer Pflanze, ausgewählt unter *Ilex aquifolium, Ilex arnhemensis, Ilex canariensis, Ilex chinensis, Ilex crenata, Ilex mitis, Ilex opaca, Ilex paraguariensis, Ilex perado* und *Ilex vomitoria,* wobei der genannte Extrakt unter den Glyzerin-, Hydroglyzerin- und Glyzerin-Hydroalkoholextrakten ausgewählt wurde, und der Gehalt am genannten Extrakt in der Zubereitung zwischen 0,5 und 5 Gewichtsprozent bezogen auf das Gesamtgewicht der Zubereitung liegt, als Kosmetikwirkstoff zur Bleichung/Depigmentierung der Haut.

8. Gebrauch nach Patentanspruch 7, **dadurch gekennzeichnet, dass** das genannte Mittel beabsichtigt, lichtbedingten oder chronologischen Alterserscheinungen der Haut vorzubeugen und/oder sie zu behandeln.

9. Nicht-therapeutisches Verfahren zur Bleichung/Depigmentierung eines Bereiches menschlicher Haut, einen Schritt der örtlichen Anwendung einer wirksamen Menge der Zubereitung nach irgendeinem der Patentansprüche 1 bis 5 auf den genannten Ziel-Hautbereich einer Person umfassend.

## Claims

1. A composition of at least one seed extract of a plant chosen from *Ilex aquifolium, Ilex arnhemensis, Ilex canariensis, Ilex chinensis, Ilex crenata, Ilex mitis, Ilex opaca, Ilex paraguariensis, Ilex perado* and *Ilex vomitoria* ; which extract is chosen from glycerinated, hydroglycerinated and hydroalcoholic glycerinated extracts; and the content of seed extract of a plant belonging to the genus *Ilex* in the composition is between 0.5 and 5% by weight relative to the total weight of the composition.

2. The composition of the claim 1, **characterized in that** the plant of the genus *Ilex* is *Ilex aquifolium.*

3. The composition according to any one of claim 1 or 2, **characterized in that** the seed extract of a plant of the genus *Ilex* is a glycerinated extract.

4. The composition according to any one of claim 1 to 3, **characterized in that** the content of seed extract of a plant belonging to the genus Ilex in the composition is between 1 and 3% relative to the total weight of the composition.

5. The composition according to any one of claim 1 to 4, **characterized in that** it further comprises at least one a desquamation agent and/or at least one other melanogenesis inhibitor.

6. A composition as defined in any one of claims 1 to 5 for use in the treatment and/or the prevention of hyperpigmentation in a subject, which hyperpigmentation is selected from post-inflammatory hyperpigmentations, hyperpigmentations with genetic determinism, hyperpigmentations of metabolic or drug origin or any other hyperpigmentation lesion.

7. Non-therapeutical use of a composition comprising at least one seed extract of a plant chosen from *Ilex aquifolium, Ilex arnhemensis, Ilex canariensis, Ilex chinensis, Ilex crenata, Ilex mitis, Ilex opaca, Ilex paraguariensis, Ilex perado* and *Ilex vomitoria* ; which extract is chosen from glycerinated, hydroglycerinated and hydroalcoholic glycerinated extracts; and the content of seed extract of a plant belonging to the genus *Ilex* in the composition is between 0.5 and 5% by weight relative to the total weight of the composition, as a cosmetic agent for the whitening/depigmentation of the skin.

8. Use according to claim 7, **characterized in that** said agent aims to prevent and/or treat photo-induced or chronological signs of skin aging.

9. A non-therapeutical method for whitening/depigmenting of a human skin area comprising a step of topical application of an effective amount of the composition as defined in any one of claims 1 to 5 to said targeted skin area of a subject.
